Europäisches Patentamt

European Patent Office

Office européen des brevets

(19)

(11) **EP 1 452 139 A1**

(12) **EUROPEAN PATENT APPLICATION**
published in accordance with Art. 158(3) EPC

(43) Date of publication:
01.09.2004 Bulletin 2004/36

(51) Int Cl.⁷: $A61B\ 10/00$

(21) Application number: 02780055.6

(86) International application number:
PCT/JP2002/011677

(22) Date of filing: 08.11.2002

(87) International publication number:
WO 2003/039374 (15.05.2003 Gazette 2003/20)

(84) Designated Contracting States:
AT BE BG CH CY CZ DE DK EE ES FI FR GB GR
IE IT LI LU MC NL PT SE SK TR

(30) Priority: 09.11.2001 JP 2001344514

(71) Applicant: Japan Science and Technology
Agency
Kawaguchi-shi, Saitama 332-0021 (JP)

(72) Inventors:
• KANAYAMA, Naohiro
Hamamatsu-shi, Shizuoka 430-3124 (JP)
• SUMIMOTO, Kazuhiro
Kawasaki-shi, Kanagawa 216-0003 (JP)

(74) Representative: Bowman, Paul Alan
Lloyd Wise
Commonwealth House,
1-19 New Oxford Street
London WC1A 1LW (GB)

(54) **INSTRUMENT FOR MEASURING INTRAUTERINE OXYGEN METABOLISM USING OPTICAL TECHNIQUE**

(57) The detecting unit 1 of the instrument of this invention comprises optic fibers 12 and 13 for sensing the inside of the uterus by using near-infrared light of multiple wavelengths, and also comprises a sensor fixture 11 made of a duralumin plate entirely coated with a light-absorbing paint and having a shape with a curvature that allows the fixture to come in close contact with the maternal abdominal wall. This detecting unit 1 is attached to the maternal abdominal wall. The weak light signals obtained as reflections from inside the uterus are converted to electric signals, and these electric signals are arithmetically operated at the measurement control unit 2. In this manner, the inside of the uterus is continuously monitored directly or indirectly by a non-invasive method to detect the intrauterine status of Hb and $HbO_2$.

F I G. 3

(A)

(B)

**Description**

Technical Field

This invention relates to an instrument for measuring the intrauterine status of oxygenation by applying an optical technique, an instrument that is quite useful for perinatal management of the mother and baby.

Background Art

The fetus receives oxygen and nutrients from the mother's body via the uterus, the placenta, and the umbilical cord. The uterus is the environment in which the fetus grows, but the uterus cannot be directly observed, although the uterine cervix can be partly observed by way of the vagina. When the baby is born, it endures pangs of birth, i.e., periodical contractions of the uterus, which tend to be stressful to the baby especially during delivery. It has so far been difficult for the doctors to make diagnoses as to whether or not the uterus itself can supply the fetus with sufficient oxygen in the perinatal period.

Instruments that have already been developed in the past and are now in clinical use include a pulse oxymeter, which is attached to a finger or an earlobe to measure the degree of oxygen saturation by employing near-infrared light; and near-infrared spectroscopy, which has been used to monitor the intra-cerebral status of oxygenation in newborn babies. However, there has been no instrument with a sensor attached to the maternal abdominal wall to measure the oxygenation state in an organ within the abdomen and yet distant from the abdominal wall. Because it was considered impossible to measure the status of oxygenation by employing near-infrared light, no instrument has yet been developed that is able to monitor directly or indirectly the intrauterine status of oxygenation. No one has ever envisioned developing such an instrument.

Since it has been impossible to monitor the intrauterine status of oxygenation on a continuous basis, there has been no way to elucidate the mechanism in which the fetus inside the uterus falls into an underdeveloped state. The impossibility of predicting this was also inconvenient for the management of mother and baby. Therefore, it was inconceivable to perform an evaluation of the fetus, including the uterine environment, directly or indirectly from the mother's body.

Disclosure of the Invention

The instrument of this invention comprises a detecting unit (sensor) that incorporates optical fibers for sensing the inside of the uterus by using near-infrared light of multiple wavelengths, a measurement control unit that transfers and amplifies the detected signals and analyzes the dynamic state of oxygenation, a display/recording unit that records the analyzed data on the recording charts, an external output unit that transmits the data to other units, such as a data logger, and a power supply unit that supplies power to these units.

The detecting unit comprises a sensor fixture and light-emitting/receiving fibers that are connected to terminals on the fixture. The sensor fixture is made of duralumin entirely coated with a light-absorbing paint, and has a curved shape that allows the fixture to come in close contact with the mother's abdominal wall. The sensor is attached to the mother's abdominal wall. The inside of the uterus is then exposed to near-infrared light. Weak light signals are obtained at the detecting unit as reflected light and are converted to electric signals at the measurement control unit.

The measuring instrument of this invention further amplifies the electric signals and performs arithmetical operations. Thus, the instrument quantitatively monitors deoxyhemoglobin (Hb) and oxyhemoglobin (HbO$_2$) inside the uterus and continuously monitors the intrauterine status of oxygenation directly or indirectly in a non-invasive manner.

Effects of the Invention

The instrument, according to this invention, for measuring the intrauterine status of oxygenation by applying an optical technique prevents the sensor from being affected by mechanical displacement such as may be caused by uterine contractions. This can be made possible by using a metal plate to fix the light-emitting portion and thereby prevent the sensor from deviating from the light axis. In addition, the curvature of the sensor fixture can be optimized so that light is concentrated on a set position inside the uterus under the maternal abdominal wall. This enable the precise measurement of the intrauterine status of oxygenation.

Furthermore, continuous monitoring has made it possible to elucidate the mechanism of fetal growth retardation in the uterus. In high-risk cases experienced in the past, it was difficult to determine the cause or sort out such problems as whether the retardation is attributable to the mother's body, the fetus itself, or the placenta. Because of continuous monitoring, doctors are now able to sort out cases of unknown causes and diagnose some cases as having problems clearly in the fetal environment in the uterus, including the uterine muscles, fetus, and placenta. Problems can be further clarified by employing an ultrasonic diagnostic device to diagnose such cases as whether the site of the sensor attachment is filled with much amniotic fluid, whether the site is near the placenta, or whether the site is near the fetus. The instrument of this invention can also be applied to predict some cases in which the fetus is distressed during delivery, and thus it is quite useful for the management of the mother and baby.

Brief Description of the Drawings

Figure 1 is a diagram showing the main configuration of the instrument of this invention.

Figure 2 is a graph showing the relationships between the wavelengths of near-infrared light and the absorption coefficients for Hb and $HbO_2$.

Figures 3(A) and 3(B) are schematic diagrams showing the configuration of the detecting unit (the sensor) of this invention.

Figure 4 is a flow diagram showing the measurement procedure used in this invention.

Figures 5(A) and 5(B) are output waveform charts showing the relationships between wavelengths and $HbO_2$, Hb, heart rate of the fetus, and uterine contractions.

Preferred Embodiment of the Invention

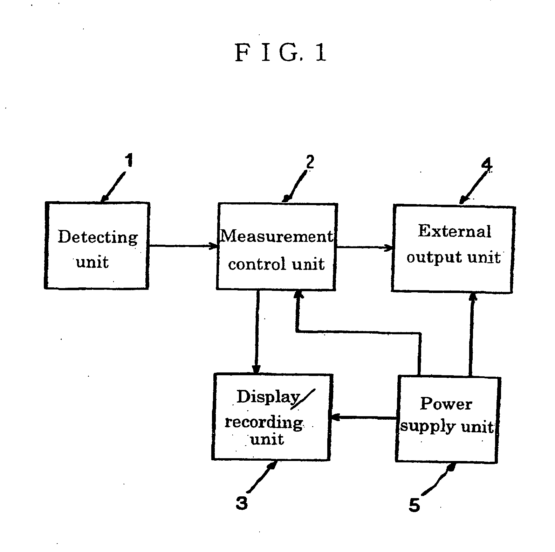

This invention is further described with respect to its preferred embodiment, referring to Figs. 1 to 5.

As shown in Fig. 1, the instrument of this invention comprises a detecting unit 1 (sensor) for sensing the inside of the uterus by using near-infrared light; a measurement control unit 2 that has photoelectric transfer elements, which amplifies and arithmetically operates on the detected signals, and analyzes the dynamic status of oxygenation; a display/recording unit 3 that records the analyzed data on recording charts; an external output unit 4 that transmits the data to other devices, such as a data logger; and a power supply unit 5 that supplies power to these units. The sensor on the detecting unit 1 is made of optical fibers, is fitted directly to the uterine cervix or indirectly to the maternal abdominal wall, and emits near-infrared light. The near-infrared light used had 4 wavelengths in the range of 775 to 904 nm.

The near-infrared light emitted by the light-emitting fibers is irradiated onto the maternal abdominal wall, and the reflected light is detected by the light-receiving fibers at the detecting unit 1. The weak light signals obtained are converted to electric signals by the photoelectron multiplier of the measurement control unit 2. The converted electric signals are further amplified and arithmetically operated on in this unit. The levels of deoxyhemoglobin (Hb) and oxyhemoglobin ($HbO_2$) in the uterine muscle layers are displayed on the liquid crystal screen of the display/recording unit 3. The data are also recorded continuously on recording charts.

More particularly, the absorbance of Hb and $HbO_2$ can be obtained by utilizing the Beer-Lambert Law. Using OD as the absorbance; a, as the absorption coefficient, C, as the concentration of the substance; L, as the actual distance; B, as the coefficient for obtaining the light path length; and G, as the constant based on the shape of the substance, the following equation is formulated:

$$OD = (a \cdot c \cdot L \cdot B) + G \qquad (1)$$

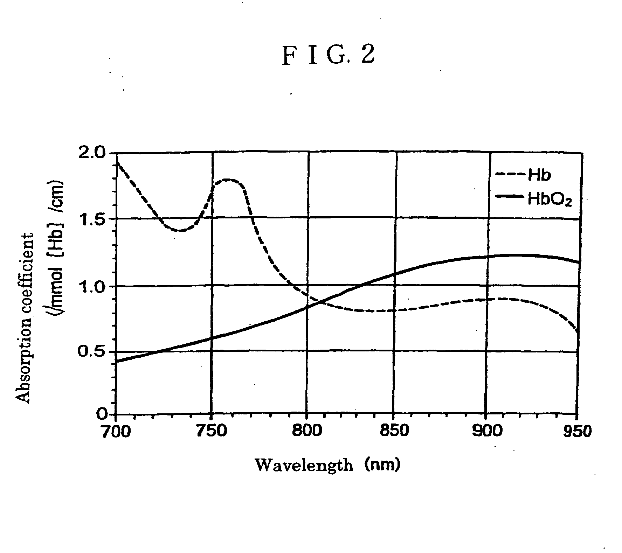

The absorption coefficients for deoxyhemoglobin (Hb) and oxyhemoglobin ($HbO_2$) at various wavelengths of near-infrared light are obtained by exposing biological tissues to the near-infrared light roughly in the range of 700 to 950 nm, and by measuring the changes in absorbance. Figure 2 shows the absorption coefficient ($\alpha$) for Hb with a dotted line and the absorption coefficient ($\beta$) for $HbO_2$ with a solid line.

The relationships between the respective wavelengths and absorption coefficients shown in Fig. 2 had been saved beforehand in the memory of the operating device (microcomputer) of the measurement control unit 2. Therefore, if B, L, and G are constant, then $\Delta c$, the changes in concentrations of deoxyhemoglobin (Hb) and oxyhemoglobin ($HbO_2$), can be obtained from the following equation:

$$\Delta c = \Delta OD/(a \cdot L \cdot B) \qquad (2)$$

If $\Delta X$ represents the change in Hb concentration; and $\Delta Y$, the change in the concentration of $HbO_2$ under the condition that deoxyhemoglobin (Hb) and oxyhemoglobin ($HbO_2$) exist in a mixed state, then the following equation is formulated for the change in absorbance, $\Delta OD$:

$$\Delta OD = \alpha \cdot \Delta X + \beta \cdot \Delta Y \qquad (3)$$

When the maternal abdominal wall is exposed to near-infrared light of, e.g., 775 nm during actual measurement, and since at that time, $\alpha_1$ represents the absorption coefficient for Hb, and $\beta_1$ represents the absorption coefficient for $HbO_2$, $\Delta OD_1$, the change in absorbance, is given by the following equation:

$$\Delta OD_1 = \alpha_1 \cdot \Delta X + \beta_1 \cdot \Delta Y \qquad (4)$$

Then, the maternal abdominal wall is exposed to near-infrared light of, e.g., 825 nm. Since at that time, $\alpha_2$ represents the absorption coefficient for Hb, and $\beta_2$ represents the absorption coefficient for $HbO_2$, the following equation is given for the change in absorbance, $\Delta OD_2$:

$$\Delta OD_2 = \alpha_2 \cdot \Delta X + \beta_2 \cdot \Delta Y \qquad (5)$$

Although $\Delta X$ and $\Delta Y$, the changes in the concentrations of Hb and $HbO_2$, can be obtained from these two equations (4) and (5), further measurements are made in this invention at 2 more wavelengths of, e.g., 850 nm and 904 nm. Using the detected data from the measurements at 4 wavelengths as 1 cycle, the operating device of the measurement control unit 2 makes calculations based on the least squares method instantaneously, and gives $\Delta X$ and $\Delta Y$, the changes in the respective concentrations of Hb and $HbO_2$. Similar measurements and calculations are made continuously along the time axis.

Once these changes, $\Delta X$ and $\Delta Y$, are available for Hb and $HbO_2$, the data are expressed in the waveforms depicted along the time axis on the liquid crystal screen of the display/recording unit 3. It is also possible to record the data continuously on recording charts. In addition, the results of the arithmetic operations are transmitted through the external output unit 4 to a data logger and other devices, including personal computers.

The measurement results indicate that the absorbance differs depending on whether or not hemoglobin is combined with oxygen. When the changes in absorbance are extracted as signals, it is possible to monitor the status of oxygenation in the uterus (including the uterine muscles, fetus, and placenta) using these signals. The instrument of this invention allows the doctors to make diagnoses of the oxygenation status of the fetus in the uterus. It becomes possible to make earlier evaluations than those possible when the doctor diagnoses the fetal condition from changes in heart rate caused by central nervous control as detected by way of the placenta.

Usually, when the uterus is contracted, the change in its shape creates a displacement through the maternal abdominal wall. A conventional parturiometer utilizes this displacement to detect conventional labor signals. However, the measuring instrument of this invention utilizes the absorbance of near-infrared light to measure the status of hemoglobin oxygenation. In such an instrument, it is important to avoid the influence of this displacement so that precise data can be obtained from the measurements.

The instrument for measuring the intra-cerebral status of oxygenation in newborn babies utilizes a sensor fixed to the baby's head. Once the sensor is attached, there is no displacement caused over time. If the sensor attached to the maternal abdominal wall is to be protected against displacement caused by uterine contractions during delivery, the sensor must be protected from any changes in the light axis by employing a metal plate or similar means. In addition, it is necessary to optimize the curvature of the sensor fixture so that the light is effectively concentrated on the uterine muscular strata under the maternal abdominal wall.

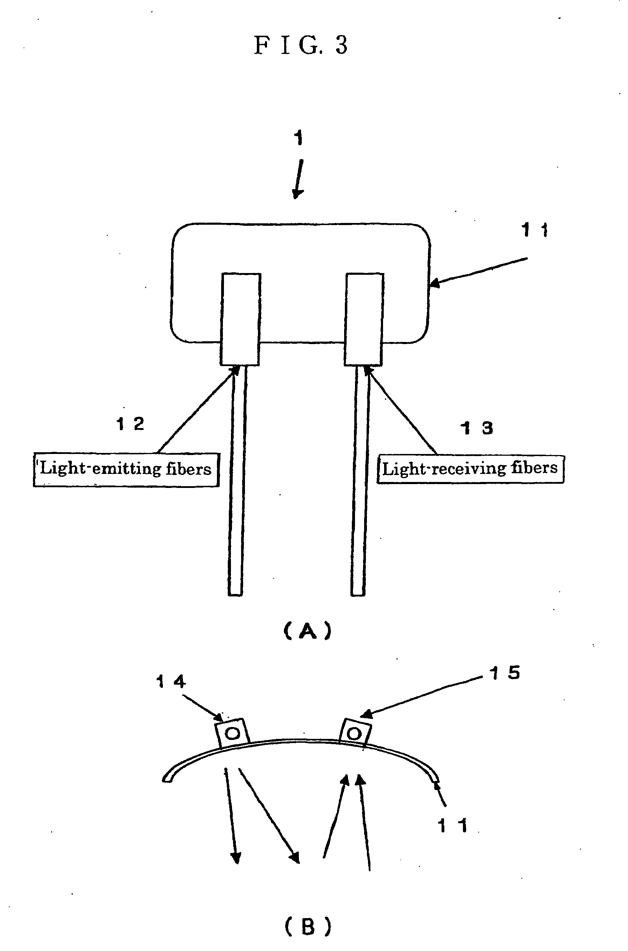

In order to protect the sensor from the effects of mechanical displacement such as may be caused by uterine contractions, the inventors have devised a means to minimize the changes in the light axis by fixing the light-emitting/receiving unit. It has been confirmed that the status of oxygenation can be monitored precisely. Figure 3 shows an embodiment of the sensor fixture that has been designed according to this concept. Figure 3(A) is a front view and Fig. 3(B) is a side view of the sensor.

The sensor fixture used in this invention is described, referring to Figs. 3(A) and 3(B). The sensor 1 comprises a sensor fixture 11, light-emitting fibers 12 and light-receiving fibers 13. The sensor fixture 11 is made of duralumin. Terminals 14 and 15 are fabricated by cutting out the duralumin and mounting them on the fixture with screws. The light-emitting fibers 12 are connected to terminal 14; and the light-receiving fibers 13, to terminal 15.

The sensor fixture 11 is entirely coated with a light-absorbing paint. It is formed in a shape with a curvature that allows the fixture to come in close contact with the maternal abdominal wall. Double-faced adhesive tape is used to attach the sensor fixture 11 to the abdominal wall. Thus, it has become possible to measure precisely the intrauterine status of oxygenation from the maternal abdominal wall.

Adjustments must be skillfully made between this sensor portion, the strength of the emitted light enough to be able to reach the uterine muscular strata, and the sensitivity of the light-receiving system where the weak signals carrying the status of hemoglobin oxygenation are amplified. As a result, the intrauterine status of oxygenation can be monitored continuously in a non-invasive manner.

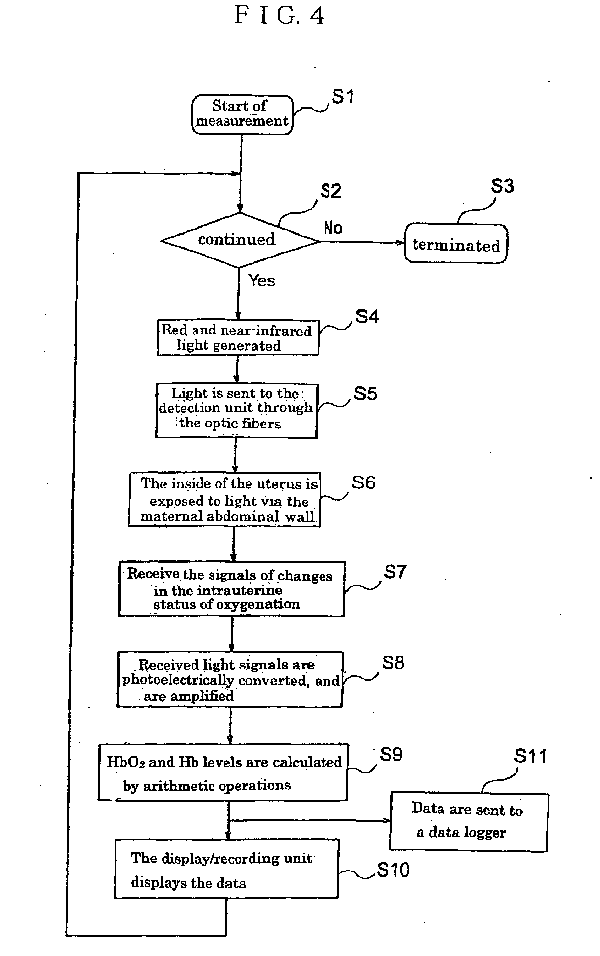

Following the flow diagram for the measuring procedure shown in Fig. 4, a description is now given as to how the intrauterine status of oxygenation is measured by employing the instrument of this invention. In Step One (S1), the measurement starts. In Step Two (S2), whether or not the measurement is continued is decided. If the measurement is not continued, then it is terminated in Step Three (S3).

If it is decided in Step Two (S2) that the measurement is continued, then in Step Four (S4), a means for emitting near-infrared light (not shown) emits the light of a given wavelength (e.g., 775 nm). In Step Five (S5), the light is sent to the detecting unit (sensor) 1 through the light-emitting fibers. In Step Six (S6), the uterus is exposed, via the maternal abdominal wall, to the near-infrared light coming through the light-emitting fibers 12.

In Step Seven (S7), the light-receiving fibers 13 receive the light signals that show the changes in the intrauterine status of oxygenation, i.e., the signals of changes in absorbance. In Step Eight (S8), the measurement control unit 2 converts the received light signals to electric signals by means of photoelectric transfer elements.

In Step Nine (S9), the measurement control unit 2 refers to the data already saved in the memory to obtain α and β, the absorption coefficients for Hb and $HbO_2$ at various wavelengths, from Fig. 2 which shows the relationships between the wavelengths and absorption coefficients for Hb and $HbO_2$. Then, ΔX and ΔY are calculated to obtain the changes in the concentrations of deoxyhemoglobin (Hb) and oxyhemoglobin ($HbO_2$).

For example, the maternal abdominal wall is exposed to near-infrared light at wavelengths of 775 nm, 825 nm, 850 nm, and 904 nm through the optic fibers. ΔX and ΔY, the respective changes in concentrations corresponding to the absorption coefficients, α and β, are obtained from equation (1). With the values measured at these 4 wavelengths used as 1 cycle, the arithmetic operation means of the measurement control unit 2 instantly calculates the correct values of ΔX and ΔY, the changes in concentrations of Hb and $HbO_2$. Similar measurements and calculations are carried out continuously along the time axis.

In Step Ten (S10), ΔX and ΔY, the changes in the respective concentrations of Hb and $HbO_2$, are displayed on the liquid crystal screen of the display/recording unit 3 in waveforms corresponding to the changes along the time axis. The data can also be recorded continuously on recording charts using a printer. If necessary, the results of these calculations can be transmitted in Step Eleven (S11) from the external output unit 4 to a data logger or other instruments, including a personal computer. Then, the procedure returns again to Step Two (S2), and the next cycle of measurements is repeated, starting with the measurements under the near-infrared light of 4 wavelengths.

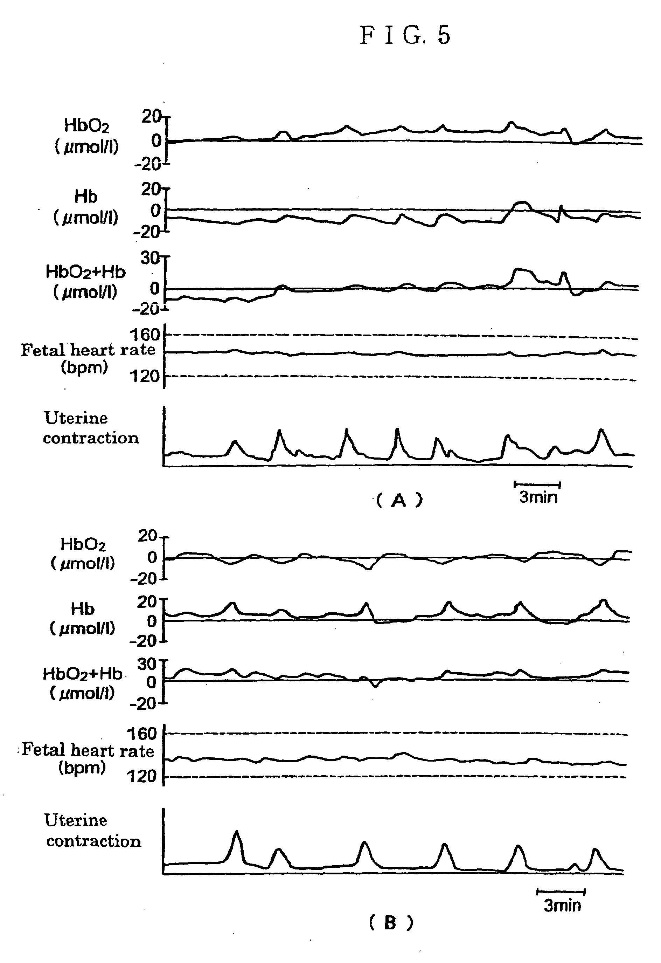

Figures 5(A) and 5(B) show the results thus obtained, which are output as graphs of waveforms, indicating the relationships between the wavelengths of near-infrared light and $HbO_2$, Hb, fetal heart rate, and uterine contractions. Figure 5(A) shows increases in the levels of $HbO_2$ and Hb and in the cerebral blood flow rate ($HbO_2$ + Hb) during contractions in cases where the uterine contractions are followed by a slight increase in the fetal heart rate. Figure 5(B) shows an increase in the Hb level and a decrease in the $HbO_2$ level, and an overall slight increase in the total hemoglobin ($HbO_2$ + Hb).

After the oxygen saturation was measured for the uterine cervix, it was found that, as far as the oxygen environment is concerned, the level in the uterine muscular strata was lower than in the limbs or the earlobe. When the sensor was attached to the maternal abdominal wall for continuous monitoring, it was also found that the level of oxyhemoglobin rises in response to the stress of uterine contractions even in normal cases.

Decreases in oxyhemoglobin and decreases in deoxyhemoglobin were observed in cases in which the fetus was in poor condition because the umbilical cord was wound around the neck or shoulders of the fetus, thus hindering fetal blood circulation. When more case data can be collected, it may be possible to make diagnoses as to whether the uterine environment is good or bad, not only during delivery but also during or before pregnancy. Thus, it will become possible to evaluate objectively the fetal environment including the uterus and to obtain a diagnostic means and instrument that are quite useful in the perinatal management of mother and child.

Industrial Applicability

As obvious from the foregoing description, the instrument of this invention for measuring the intrauterine status of oxygenation using an optical technique makes it possible to measure the status of oxygenation in the uterus (including uterine muscles, fetus, and placenta) in a non-invasive (non-painful) manner. Since the measurements can be monitored continuously, it will become possible to elucidate the mechanism of fetal growth retardation in the uterus, which has been an unexplained phenomenon in the past. The instrument of this invention will also make growth retardation predictable in the future. Doctors will be able to diagnose cases of growth retardation as having been caused by factors relating to the intrauterine environment. In total, the instrument of this invention is an effective means that is quite useful in the perinatal management of mother and child.

**Claims**

1. An instrument for measuring the intrauterine status of oxygenation by applying an optical technique, which comprises:

   a detecting unit for sensing the inside of the uterus by using near-infrared light,
   a measurement control unit that amplifies the detected signals coming from said detecting unit, makes arithmetic operations, and analyzes the dynamic state of oxygenation,
   a display/recording unit that deals with the analyzed data,
   an external output unit that transmits the data to other devices, such as a data logger, and
   a power supply unit that supplies power to these units,

   wherein said detecting unit is provided with a sensor fixture, light-emitting fibers and light-receiving fibers, with both fibers being fitted to their respective terminals formed on said sensor fixture,
   wherein said sensor fixture is made of a duralumin plate coated entirely with a light-absorbing paint, and is of a shape that has a curvature which enables the fixture to come in close contact with the maternal abdominal wall, and
wherein the sensor is attached directly to the uterine cervix or indirectly to the maternal abdominal wall so that the inside of the uterus can be exposed to the near-infrared light.

F I G. 1

EP 1 452 139 A1

F I G. 2

F I G. 3

1

11

12

Light-emitting fibers

13

Light-receiving fibers

(A)

14

15

11

(B)

# F I G. 4

```
                    Start of        S1
                   measurement


                      │
                      │
                      ▼
                                    S2              S3
                    ◇ continued ◇   No   ──────▶  terminated
                      │
                      │ Yes
                      ▼
              Red and near infrared    S4
                 light generated
                      │
                      ▼
              Light is sent to the     S5
            detection unit through
               the optic fibers
                      │
                      ▼
            The inside of the uterus is   S6
             exposed to light via the
             maternal abdominal wall
                      │
                      ▼
           Receive the signals of changes   S7
              in the intrauterine
             status of oxygenation
                      │
                      ▼
             Received light signals are     S8
          photoelectrically converted, and
               are amplified
                      │
                      ▼
          HbO2 and Hb levels are calculated   S9        S11
            by arithmetic operations
                      │                          Data are sent to
                      ├──────────────────────▶   a data logger
                      ▼
            The display/recording unit
              displays the data        S10
```

# F I G. 5

( A )

3min

( B )

3min

<table>
<tr><td colspan="2" align="center">**INTERNATIONAL SEARCH REPORT**</td><td>International application No.<br>PCT/JP02/11677</td></tr>
</table>

| A. CLASSIFICATION OF SUBJECT MATTER |
|---|
| Int.Cl⁷ A61B10/00 |

According to International Patent Classification (IPC) or to both national classification and IPC

| B. FIELDS SEARCHED |
|---|
| Minimum documentation searched (classification system followed by classification symbols)<br>Int.Cl⁷ A61B10/00 |

Documentation searched other than minimum documentation to the extent that such documents are included in the fields searched

| Jitsuyo Shinan Koho | 1922–1996 | Toroku Jitsuyo Shinan Koho | 1994–2002 |
|---|---|---|---|
| Kokai Jitsuyo Shinan Koho | 1971–2002 | Jitsuyo Shinan Toroku Koho | 1996–2002 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| Y | WO 99/40842 A1 (Non-Invasive Technology, Inc.),<br>19 August, 1999 (19.08.99),<br>& JP 2002-502654 A | 1 |
| Y | JP 9-135825 A (Hitachi, Ltd.),<br>27 May, 1997 (27.05.97),<br>Par. No. [0019]<br>(Family: none) | 1 |
| Y | WO 00/57793 A1 (Hitachi Medical Corp.),<br>05 October, 2000 (05.10.00),<br>Par. No. [0033]<br>(Family: none) | 1 |

| ☐ Further documents are listed in the continuation of Box C. | ☐ See patent family annex. |
|---|---|

| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
|---|---|

| Date of the actual completion of the international search<br>03 December, 2002 (03.12.02) | Date of mailing of the international search report<br>17 December, 2002 (17.12.02) |
|---|---|
| Name and mailing address of the ISA/<br>Japanese Patent Office | Authorized officer |
| Facsimile No. | Telephone No. |

Form PCT/ISA/210 (second sheet) (July 1998)